# EUROPEAN PATENT SPECIFICATION

(11) **EP 0 293 889 B1**
(45) Date of publication and mention of the grant of the patent: **13.04.1994**
(21) Application number: 88108826.4
(22) Date of filing: 01.06.1988
(51) Int. Cl.: C08G 59/40

(54) **Fluorinated epoxy resins and process for preparing them**
Fluorierte Epoxy-Harze und Verfahren für deren Herstellung
Résines époxydes fluorées et méthode pour leur préparation

(30) Priority: 02.06.1987 IT 2074787
(43) Date of publication of application: 07.12.1988
(73) Proprietor: AUSIMONT S.p.A., I-20121 Milano (IT)
(72) Inventor: Re, Alberto, I-20129 Milan (IT); Tonelli, Claudio, I-20049 Concorezzo (IT); Tortelli, Vito, I-20137 Milan (IT)
(74) Representative: Barz, Peter, Dr.

(56) References cited:
- EP-A- 0 212 319
- EP-A- 0 230 112
- EP-A- 0 249 048
- CHEMICAL ABSTRACTS, vol. 81, no. 6, 12th August 1974, page 58, abstract no. 26679a, Columbus, Ohio, US; & JP-A-74 27 543

## Description

The present invention relates to fluorinated epoxy resins and to a process for preparing them starting from non-fluorinated epoxy resins of a known type, which are cross-linked with fluorinated polyfunctional cross-linking agents comprising a chain of fluoroalkylene units. The cross-linked resins thus obtained have improved properties as compared with the corresponding epoxy resins not containing fluorine.

EP-A-212 319 discloses inter alia the curing of non-fluorinated epoxy resins with perfluorooxyalkene-diols wherein the alkylene chain comprises units with one carbon atom. The cured resins show good surface properties, resistance to hydrolysis and a low dielectric constant.

The specific characteristics of the cross-linked resins obtained in accordance with the present invention are:
- surface properties which impart oil and water repellence and a low friction coefficient;
- resistance to hydrolysis and minimum absorption of water;
   resistance to solvents;
- advantageous dielectric characteristics, in particular as regards the dielectric constant and the volume resistivity.

The present invention is based on the surprising fact that, starting from a non-fluorinated epoxy resin and cross-linking said resin with a polyfunctional fluoroalkane, it is possible to obtain a cross-linked product having the typical characteristics of a cross-linked epoxy resin, which has been obtained starting from a fluorinated epoxy polymer.

The cross-linking process according to the invention as defined in the claims provides cross-linked products which, although they have a fluorine content not exceeding 15% and, preferably, ranging from 1% to 5% by weight, exhibit the same typical surface characteristics as the cross-linked epoxy resins having a high fluorine content, which results in high oil and water repellence, a minimum absorption of water and a high resistance to hydrolysis and to solvents.

Specific examples of starting epoxy resins are:
- Epoxy resins from bisphenol A - epichlorohydrin 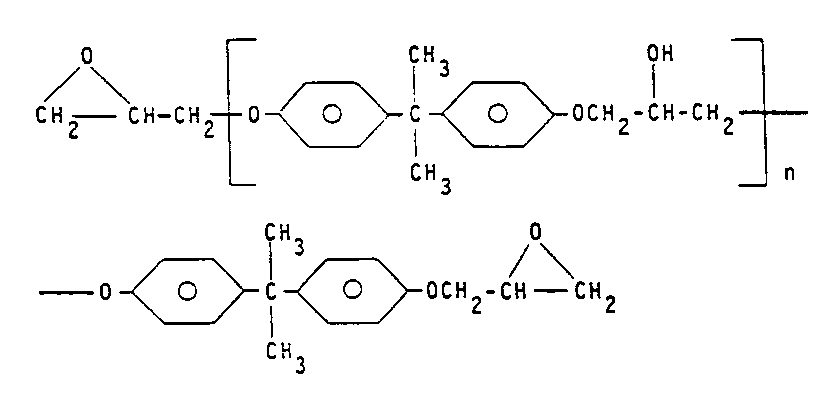
- Epoxy cresol novolak resins 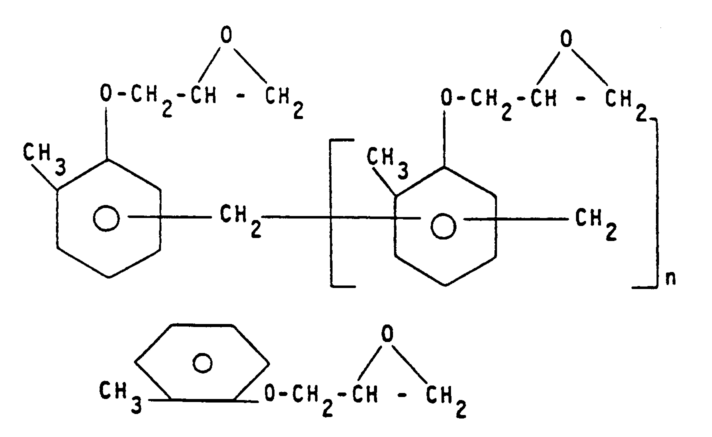
- Epoxy phenolic novolak resins 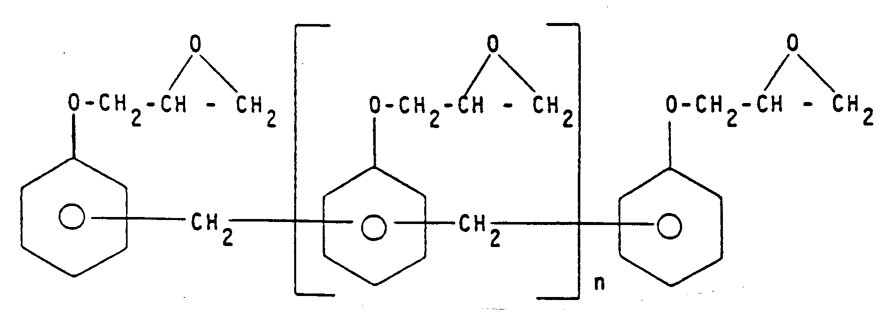
- Resins from bisphenol F

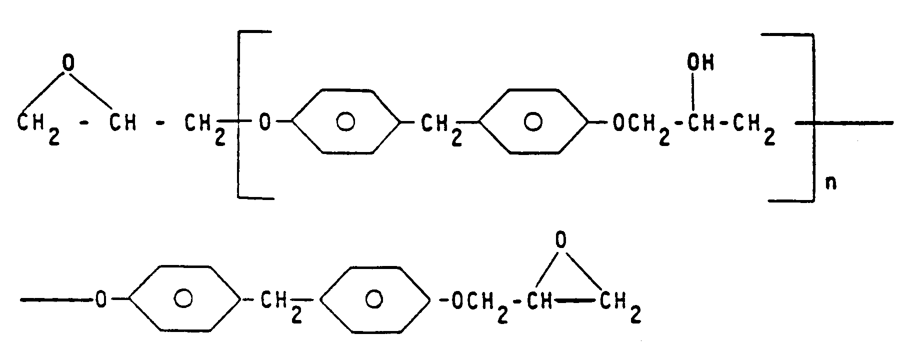

In the above formulae, n has a value ranging from 1 up to 10 and above (see Kirk Othmer, Encyclopedia of Chemical Technology, Vol. 8, Ed. 1967, page 300).
- Polynuclear resins from phenol - glycidyl ether 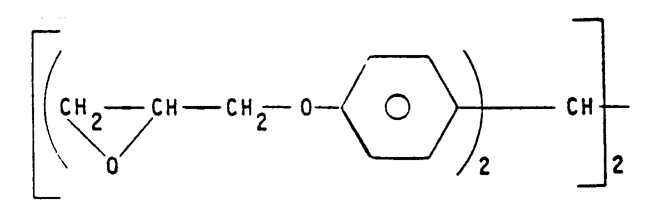
- Resins from tetraglycidylmethylene dianiline 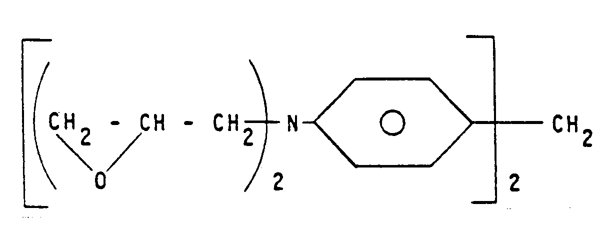
- Resins from triglycidyl isocyanurate 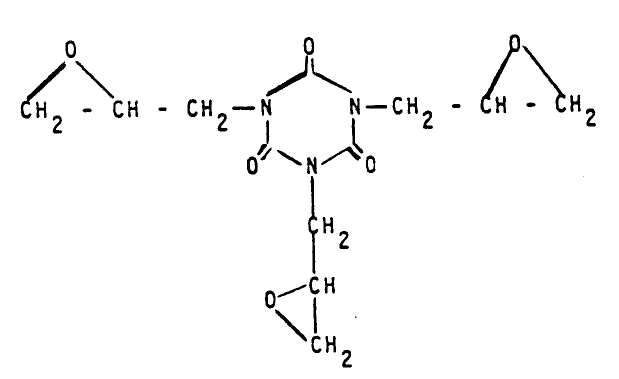
- Resins from diglycidylhydantoin 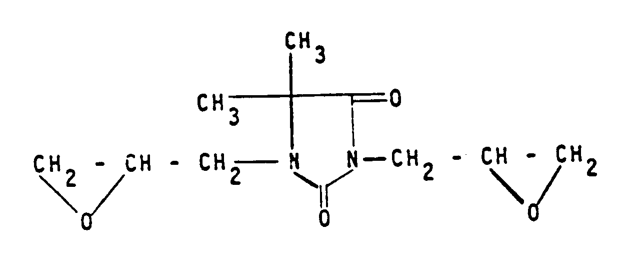
- Resins derived from bisphenols or halogenated bisphenol monomers for example, tetrabromobisphenol A and tetrachlorobisphenol A.

It is also possible to use mixtures of one or more of the above-mentioned resins.

The cross-linking agent used in the present invention is represented by a perfluoroalkane having a straight or branched structure and functional groups at both ends which are reactive with the starting epoxy resin and are selected from NCO, NH₂, COOH, OH, SH and anhydride groups.

The cross-linking agent has one of the following formulae:

I) A-(CF₂CF₂)ₙ-A'

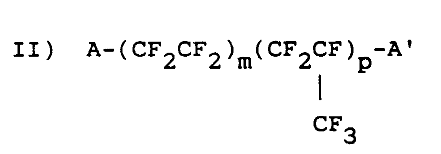

wherein n = 2-8; m = 1-8; p = 0-6; m + p being at least 2; A and A′ are end groups containing two or more of the functional groups mentioned above.

End groups A and A′ may optionally contain a divalent radical W linking the functional group and the perfluoroalkylene chain. The linking divalent radical W may be of the aliphatic, cycloaliphatic, aromatic or heterocyclic type. Linking groups are, for example:
W.1) - (CH₂)ₘ- wherein m = 1 or 2; 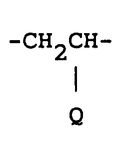 wherein Q is C₁-C₃ alkyl or phenyl; 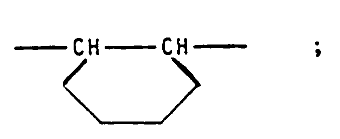 -(CH₂)ₘ(OCH₂CH₂)ₛ- wherein s = 1-8 and m =1 or 2;
   -(CH₂)ₘ--CHCH₃-; 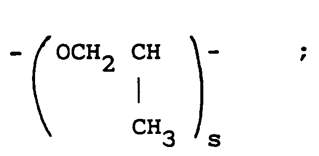
   -OCH₂CH₂CH₂-.

Group W can, furthermore, be selected from
W.2) arylene radicals such as: 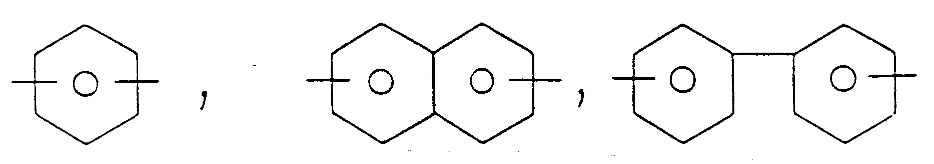 optionally also containing substituents on the ring,
W.3) heterocyclic radicals such as 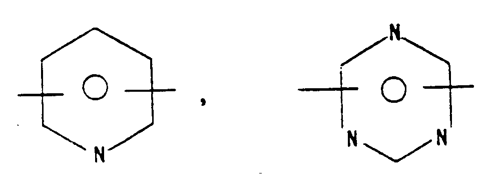
W.4) or polycyclic radicals such as

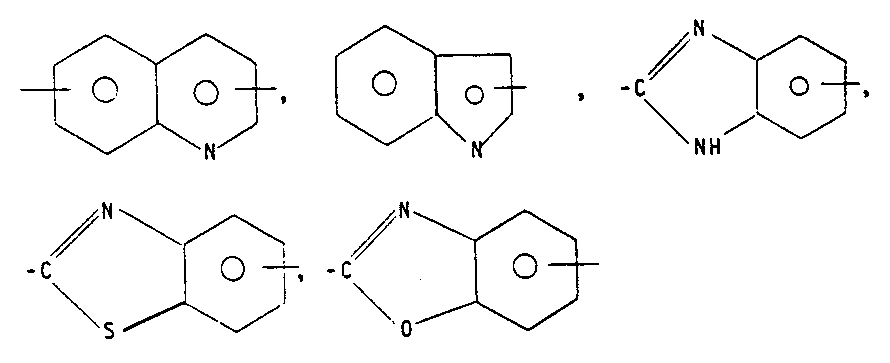

The linking groups of type W.2), W.3) and W.4) can be bound to the perfluoroalkylene chain either directly or through one or two -CH₂- groups.

The cross-linking agent can also have the functional groups on only one end of the perfluoroalkylene chain, the other end group being free of functional groups.

The following polyfunctional end groups may, for example, be used:
-(CH₂CH₂OH)₂; 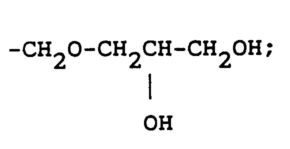
diisocyanates, 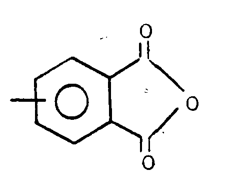
and other anhydride end groups susceptible to ring opening under the reaction conditions.

The preparation of polyfunctional perfluoroalkanes to be used according to the present invention is, in particular, described in IT-A-19779 A/86 and 19780 A/86.

When practising the present invention, the fluorinated cross-linking agent according to the invention can also be used in admixture with non-fluorinated cross-linking agents of known type. This permits maintenance of the fluorine content in the above-indicated range.

Depending on the field of use, the epoxy resins utilizable for the present invention may be liquid, solid or in solution. The liquid epoxy resins predominantly contain epoxy groups and are cross-linked with reagents which contain active hydrogens, for example polyamines, polycarboxylic acids, polythiols, polyphenols, polyaminoamides or anhydride groups, optionally in the presence of catalysts such as acids or Lewis bases, for example, complexes of boron trifluoride or tertiary amines.

In accordance with the present invention, the liquid epoxy resins are cross-linked with perfluoroalkane derivatives containing end groups as defined in the claims such as, for example, NH₂, COOH, (CO)₂O, SH.

The cross-linking reaction may, for example, be conducted at room temperature (with a non-aromatic amine), up to temperatures higher than 200°C (with an anhydride).

The conventional technique for the cross-linking of epoxy resins with cross-linking agents of the art is fully utilizable in the present invention.

The solid epoxy resins contain epoxy end groups and free hydroxy groups in the chain. Thus, in accordance with the present invention, they can be cross-linked with the above-mentioned reagents or by reaction with a fluoroalkane having an isocyanate end group. In this case, the reaction giving rise to the formation of polyurethane bonds can be catalyzed or not by the catalysts which are generally utilized in the technology of the polyurethanes obtained from polyols and polyisocyanates. In any case, the addition of an efficacious catalyst system permits operation at low temperatures (20 to 100°C) and for short times. Furthermore, a proper amount of added catalyst permits optimization of the pot life, i.e., the time during which the reaction mixture remains sufficiently fluid.

As catalysts it is possible to employ tin derivatives such as dibutyl tin dilaurate, dibutyl tin diacetate, dibutyl tin oxide, iron derivatives such as iron acetylacetonate, titanium alcoholates such as titanium tetraisopropylate and tertiary amines such as triethylamine. The amounts range from 0.001 to 2% by weight, preferably from 0.01 to 0.5%, based on the total weight.

Generally, the admixing of the fluorinated cross-linking agent does not give rise to particular problems. In some cases it is, however, possible to facilitate the mixing by adding suitable solvents such as esters, for example, butyl acetate and amyl acetate; ketones, for example, methylethylketone and methylisobutylketone and aromatic hydrocarbons such as, for example, xylene and toluene.

The amount of solvent employed depends on the viscosity the solution is to have.

The formulations of the epoxy resins prepared in accordance with the present invention may include other ingredients such as pigments or fillers, depending on the requirements of the specific field of use, said ingredients having the purpose of reducing the costs of the manufactured articles, increasing its consistency, levelling the pigment inside the resin or of reinforcing the structure of the resin in mechanical respect.

The pigments and other fillers, being of pigment nature or not, can be added in order to coat and/or protect the surface on which the resin is spread, for example, by reflecting the destructive sun rays which, otherwise, could pass through the resin and cause a degradation of the underlying material.

Although their fluorine content is low, the resins prepared from the polymers of the invention are compatible with fillers of a particular nature such as, for example, polytetrafluoroethylene (PTFE) and C₂F₄/C₃F₆ copolymers (FEP), which may be added to improve certain mechanical characteristics such as impact strength and abrasion resistance.

The use of cross-linking agents having a fluoroalkylene chain in accordance with the present invention imparts to the conventional epoxy resins considerably improved chemico-physical and mechanical characteristics if compared with prior art hydrogenated epoxy resins since said agents render the obtainable materials suitable for a plurality of utilizations.

The resins obtainable according to the invention are, in particular, characterized by:
- a high resistance to chemical agents, to hydrolysis and to atmospheric agents,
- a high thermal resistance,
- a considerable dimensional stability,
- a low wettability,
- self-lubricating properties,
- excellent mechanical properties,
- water repellence, oil repellence,
- flame resistance
- a low dielectric constant,
- a high thermal dissipation factor.

In consideration of such exceptional characteristics, some of the applications of the products of the invention are as adhesives, structural materials and high-performance composite materials or, for example, in the sector of electronics, the use as supporting resins for printed circuits, encapsulating resins for chips, connecting resins for electric cables.

Furthermore, a very broad application field is that of coatings and paints in general and, in particular, for printed circuits, magnetic tapes and discs, optical readout discs, optical fibres and optical systems in general, paints for aeronautical and spacecraft applications, barrier paints for marine environments, hydrophobic coatings for submarine systems, coatings of mechanical parts immersed in solvents and, in general, coatings of metallic systems subject to corrosion.

Unless otherwise specified in the examples, the properties of the materials are measured according to the following procedures:
Glass transition = differential thermal analysis/heating rate = 16°C/minute
Dielectric constant (εr)/dissipation factor (Tgδ) = ASTM D 150/50 Hz/23°C.

### Example 1

22.0 g (0.10 moles) of pyromellitic anhydride in 200 ml of anhydrous acetone (distilled from pyromellitic anhydride) were charged into a 250 ml flask equipped with a cooler, a dropping funnel and a thermometer. Under magnetic stirring, the solution was brought to boiling (T = 59°C) whereafer, within about 2 hours, a solution of 19,5 g (0,05 moles) of C₆F₁₂(CH₂CH₂OH)₂ in 30 ml of anhydrous acetone was added so as to always have an excess of pyromellitic anhydride in order to prevent polymerization reactions.

After complete addition, the solution was further refluxed for 1 hour. Subsequently, acetone was entirely separated by means of a Claisen distillation apparatus. Remaining in the flask were 41 g of a white powder which, according to NMR¹⁹F and NMR¹H analyses had the following formula:

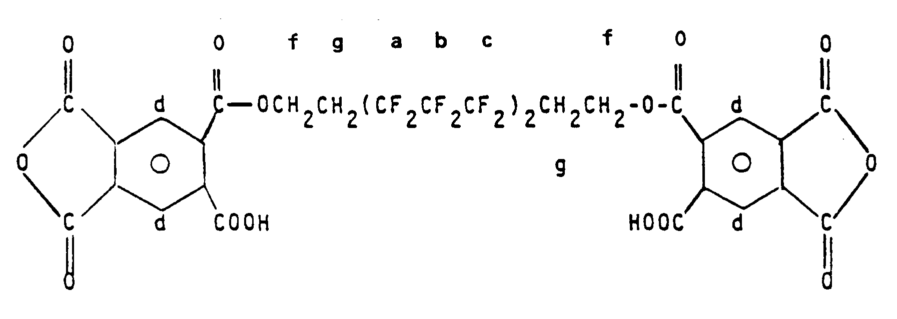

### Chemical shifts

- NMR¹⁹F: (δ, CFCl₃): a -113
b -121
c -123
- NMR¹H: (δ, TMS): d 8.0 - 8.5
f 4.7
g 2.8

In particular, the disappearance was observed of the signal at δ = 3.9, corresponding to the methylene group being in α position with respect to the hydroxy group characteristic of the starting diol.

### Example 2

Following the procedure of example 1 but using, as a fluoroalkane diol, 14.5 g (0.0050 moles) of C₄F₈(CH₂CH₂OH)₂, 36 g of 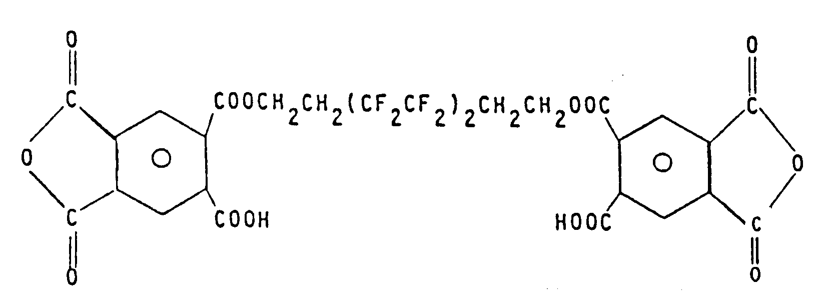
were obtained.
The structure was identified by IR and NMR analyses.

### Example 3

Following the procedure of example 1 but using, as fluoroalkane diols, 255 g (≃ 0.05 moles) of a mixture of C₈F₁₆(CH₂CH₂OH)₂ (86%) and C₁₀F₂₀(CH₂CH₂OH) (14%), 47 g of 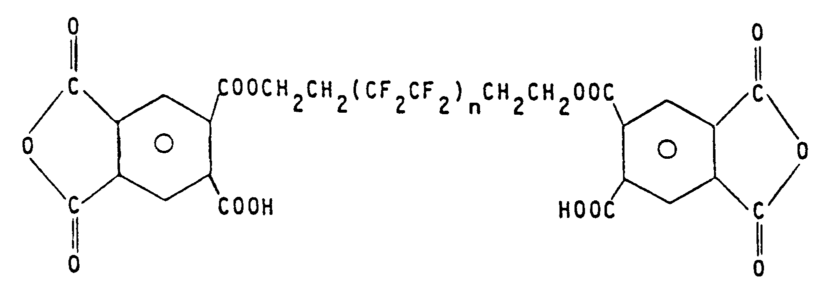
were obtained.
(n = 4 : 86%; n = 5 : 14%)
The structure was identified by IR and NMR analyses.

### Example 4

Into a 100 ml flask, in a nitrogen atmosphere, 10 g of 2,4-diisocyanatotoluene (0.057 moles) in 100 ml of diglyme were charged and, subsequently, over a period of 2 hours a solution of 7,83 g of C₄F₈(CH₂CH₂OH)₂ (0.027 moles) in 10 ml of diglyme was added. The mixture was heated to 80°C and allowed to react for 2 hours under stirring, whereafter the reaction solvent was distilled off under reduced pressure. In this manner, a white, solid product (17.2 g) was obtained, the analysis (IR, NMR) of which was in accordance with the following structure:

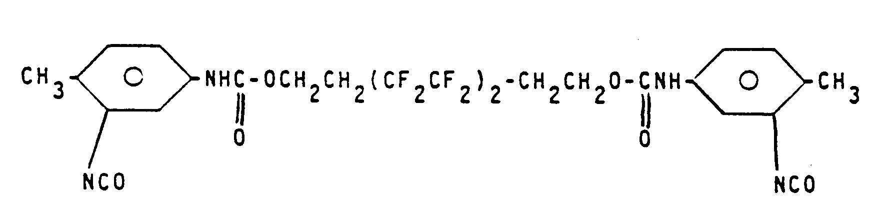

### Example 5

In a manner analogous to example 4, 12.25 g of isophorone diisocyanate (0.055 moles) were reacted with 8 g of C₄F₈(CH₂CH₂OH)₂ (0.027 moles) in diglyme.

20 g of a highly viscous, transparent liquid product were obtained, the chemical structure of which, confirmed by IR and NMR analyses was the following:

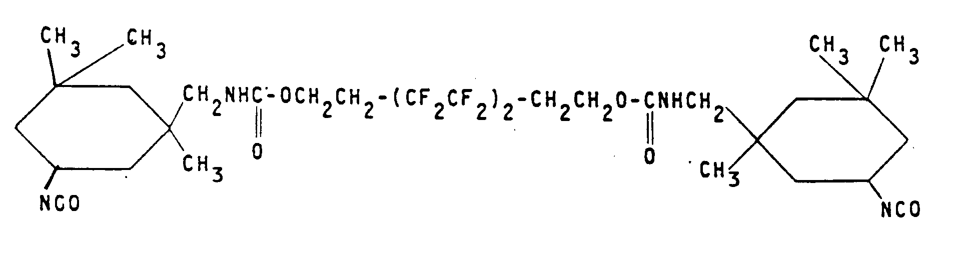

### Example 6

100 parts of a liquid epoxy resin based on bisphenol A - epichlorohydrin having an epoxy equivalent weight of 190 and 1 part of N-butyl imidazole were dissolved in acetone. An acetone solution of 90 parts of the fluorinated dianhydride 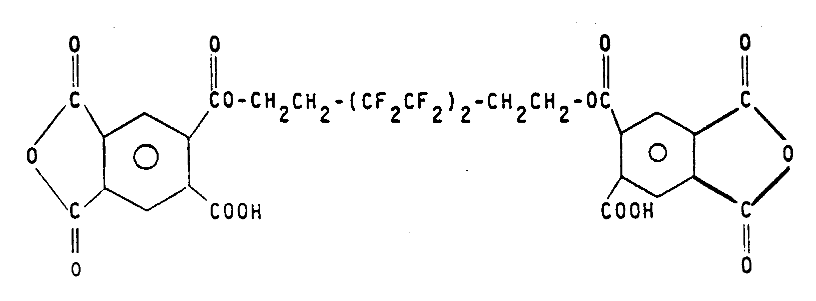
of example 2 and 1 part of N-butyl imidazole was added to this solution. The solvent was removed under vacuum and the mixture was cross-linked at 100°C for 1 hour and at 165°C for 8 hours.

The resulting resin was fully transparent, had a fluorine content equal to 10% and exhibited the following properties:

| | |
|---|---|
| Glass transition temperature: | = 156°C |
| Dielectric constant (εr) | = 3.2 |
| Water absorption | = 0.20% by weight after a 96 hour immersion at 100°C |
| Contact angle (H₂O) | = 93°. |

### Example 7

100 parts of a liquid epoxy resin based on bisphenol A - epichlorohydrin having an epoxy equivalent weight of 190 were mixed with 56.5 parts of methylnadic anhydride, 33.5 parts of the fluorinated anhydride 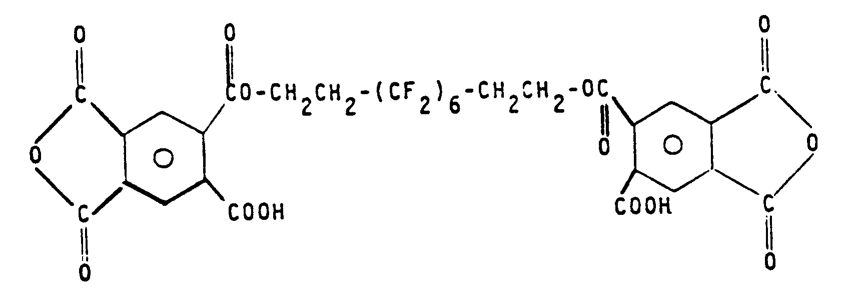
of example 2 and 1 part of N-butyl imidazole.

After deaeration under vacuum, the mixture was cross-linked at 100°C for 1 hours and at 165°C for 6 hours.

The resulting resin was fully transparent, had a fluorine content equal to 5% by weight and exhibited the following properties:

| | |
|---|---|
| Glass transition temperature | = 153°C |
| Dielectric constant | = 3.4 |
| Water absorption | = 0.35% by weight after a 96 hour immersion at 100°C |
| Contact angle (H₂O) | = 87°. |

### Example 8 (Comparative Test)

The preceding example was repeated but using only methylnadic anhydride (90 parts by weight with respect to the bisphenol A - epichlorohydrin resin). The resulting resin had a dielectric constant of 4, a water absorption of 1.7% by weight under the same conditions and a contact angle with water of 62°.

### Example 9

25 parts by weight of a solid epoxy resin based on bisphenol A - epichlorohydrin having an epoxy equivalent weight of 500, were dissolved during heating in 80 parts by weight of acetone. To this solution were added 6.5 parts of hexamethylene diisocyanate and 1.7 parts of the fluorinated diisocyanate: 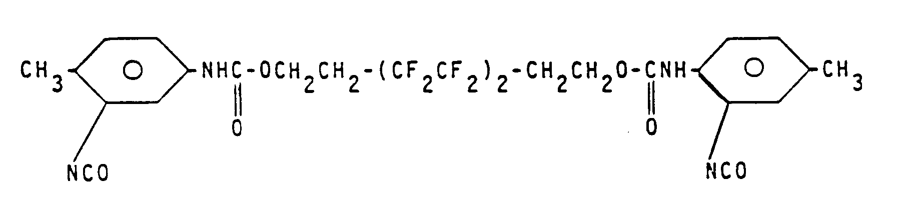
the preparation of which is described in example 4.

Acetone was evaporated and 0.002 parts by weight of an 0.2N solution of dibutyltin acetate were added. The mixture thus obtained was homogenized and cross-linked at 100°C for 6 hours. The resulting resin had a fluorine content of 1.2% by weight and exhibited the following characteristics:

| | |
|---|---|
| Dielectric constant (εr) | = 3.25 |
| Dissipation factor (Tgδ) | = 0.008 |
| Contact angle (H₂O) | = 93°. |

### Example 10

Following the procedure of example 9, a cross-linked epoxy resin was prepared starting from 20 parts of a solid epoxy resin having an epoxy equivalent weight of 500, 7.8 parts of hexamethylene diisocyanate and 2.1 parts of the fluorinated diisocyanate: 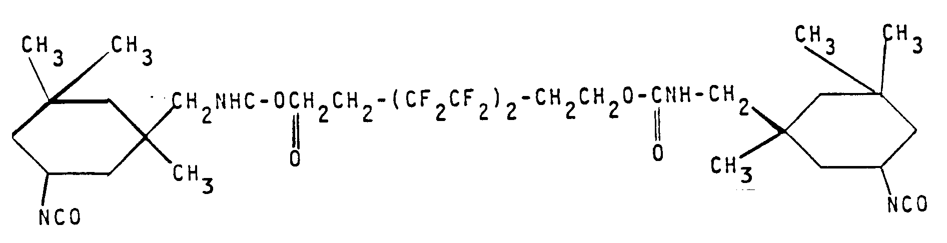
The resulting resin had a fluorine content of 1.1% by weight and the following characteristics:

| | |
|---|---|
| Dielectric constant (εr) | = 3.18 |
| Dissipation factor (Tgδ) | = 0.007 |
| Contact angle (H₂O) | = 105°. |

### Example 11

By operating as in example 10, but using a mixture of diisocyanates consisting of 5.8 parts of hexamethylene diisocyanate and 4.4 parts of the fluorinated diisocyanate, a resin was obtained which had a fluorine content equal to 2.6% by weight and exhibited the following characteristics:

| | |
|---|---|
| Dielectric constant (εr) | = 3.10 |
| Dissipation factor (Tgδ) | = 0.008 |
| Contact angle (H₂O) | = 124°. |

## Claims

1. A process for cross-linking non-fluorinated epoxy resins optionally containing hydroxy groups, characterized by using, as a cross-linking agent, a perfluoroalkane compound having one of the following formulae:
I) A-(CF₂CF₂)ₙA' 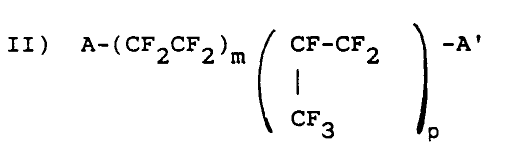
wherein:
n = 2-8; m = 1-8; p = 0-6; m + p being at least 2, A and A'are end groups containing two or more functional groups which are reactive with the starting epoxy resin and are selected from NH₂, COOH, OH, SH, NCO and an anhydride group.

2. The process of claim 1, wherein the end group A, A′ consists of one or more functional groups of the type defined in claim 1, linked to the perfluoroalkylene structure through a linking divalent radical of the aliphatic, cycloaliphatic, aromatic or heterocyclic type.

3. The process of claim 1 or 2, wherein the perfluoroalkane cross-linking agent is used in admixture with a non-fluorinated cross-linking agent.

4. Resins containing fluorine in an amount of not more than 15% by weight, preferably in an amount of from 1 to 5% by weight, obtainable by reacting non-fluorinated epoxy resins in accordance with the cross-linking process as claimed in any one of the preceding claims.

## Patentansprüche

1. Verfahren zur Vernetzung von nicht-fluorierten, gegebenenfalls Hydroxygruppen enthaltenden Epoxyharzen, gekennzeichnet durch die Verwendung, als Vernetzungsmittel, einer Perfluoralkan-Verbindung mit einer der folgenden Formeln:
I) A-(CF₂CF₂)ₙA' 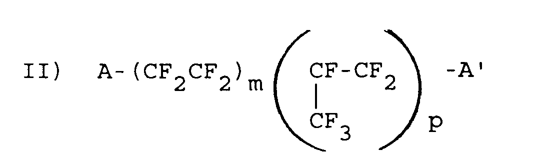
worin:
n = 2-8; m = 1-8; p = 0-6; wobei m + p mindestens 2 beträgt, A und A' Endgruppen sind, die zwei oder mehr funktionelle Gruppen enthalten, die mit dem Ausgangs-Epoxyharz reaktiv sind und aus NH₂, COOH, OH, SH, NCO und einer Anhydridgruppe ausgewählt sind.

2. Verfahren nach Anspruch 1, in welchem die Endgruppe A, A' aus einer oder mehreren funktionellen Gruppen des in Anspruch 1 definierten Typs besteht, durch einen zweiwertigen Verbindungsrest des aliphatischen, cycloaliphatischen, aromatischen oder heterocyclischen Typs an die Perfluoralkylen-Struktur geknüpft.

3. Verfahren nach Anspruch 1 oder 2, in welchem das Perfluoralkan-Vernetzungsmittel in Beimischung mit einem nicht-fluorierten Vernetzungsmittel eingesetzt wird.

4. Harze, Fluor in einer Menge von nicht mehr als 15 Gew.-%, vorzugsweise in einer Menge von 1 bis 5 Gew.-% enthaltend, erhältlich durch Umsetzung von nicht-fluorierten Epoxyharzen gemäß dem in irgendeinem der vorangehenden Ansprüche beanspruchten Vernetzungsverfahren.

## Revendications

1. Un procédé de réticulation de résines époxy non-fluorées contenant des groupes hydroxy, caractérisé en ce que l'on utilise, en tant qu'agent de réticulation, un composé perfluoroalcane présentant l'une des formules suivantes:
I) A-(CF₂CF₂)ₙ-A' 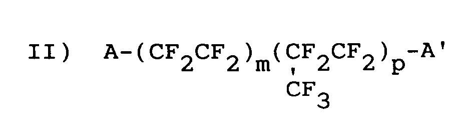
dans lesquelles:
n est compris dans un intervalle allant de 2 à 8;
m est compris dans un intervalle allant de 1 à 8;
p est compris dans un intervalle allant de 0 à 6;
m+p étant au moins égal à 2;
A et A' étant des groupes terminaux contenant au moins deux groupes fonctionnels qui réagissent avec la résine époxy de départ et qui sont choisis dans le groupe comprenant: NH₂, COOH, ON, SH, NCO et un groupe anhydride.

2. Le procédé selon la revendication 1, dans lequel les groupes terminaux A, A' sont formés d'un ou plusieurs groupes fonctionnels tels que définis dans la revendication 1, liés à la structure perfluoroalkylène par l'intermédiaire d'un radical de liaison divalent de type aliphatique, cycloaliphatique, aromatique ou hétérocyclique.

3. Le procédé selon la revendication 1 ou 2, dans lequel l'agent de réticulation perfluoroalcane est utilisé en mélange avec un agent de réticulation non-fluoré.

4. Résine contenant du fluor en une quantité qui n'est pas supérieure à 15% en poids, de préférence en une quantité de l'ordre de 1 à 5% en poids, obtenue par réaction de résines époxy non-fluorées conformément au procédé de réticulation tel que revendiqué dans l'une quelconque des revendications précédentes.
